# EUROPÄISCHE PATENTSCHRIFT

(11) **EP 2 994 166 B1**
(45) Veröffentlichungstag und Bekanntmachung des Hinweises auf die Patenterteilung: **02.08.2017**
(21) Anmeldenummer: 14723405.8
(22) Anmeldetag: 06.05.2014
(51) Int. Cl.: A61K 47/08, A61K 47/32, A61K 9/06, A61K 9/12

(54) **WÄSSRIGE GELZUBEREITUNGEN MIT DIETHYLETHER**
AQUEOUS GELS WITH DIETHYLETHER
GELS AQUEUX AVEC DIÉTHYLÉTHER

(30) Priorität: 08.05.2013 DE 102013208492
(43) Veröffentlichungstag der Anmeldung: 16.03.2016
(73) Patentinhaber: Beiersdorf AG, 20253 Hamburg (DE)
(72) Erfinder: WÖLLER, Karl-Heinz, 20257 Hamburg (DE); NIERLE, Jens, 21073 Hamburg (DE)
(86) Internationale Anmeldenummer: PCT/EP2014/059188
(87) Internationale Veröffentlichungsnummer: WO 2014/180819

(56) Entgegenhaltungen:
- EP-A1- 0 404 334
- DE-A1- 10 065 046
- DE-A1-102011 112 374

## Beschreibung

Die Erfindung umfasst Zubereitungen in Form eines Gels oder Mikroemulsion umfassend Wasser, Diethylether und Ammonium Acryloyldimethyltaurat/Vinylpyrrolidon Copolymer.

Ein weit verbreitetes Mittel zur Linderung muskuloskelettaler Schmerzzustände wie z.B. rheumatische Beschwerden, Lumbago etc. oder auch von Sportverletzungen wie z.B. Prellungen und Verstauchungen ist die äußerliche Kühlung des betroffenen Körperareals.

Die Abkühlung des Gewebes bewirkt dabei eine Analgesie durch Kälteanästhesie, eine Abnahme der Durchblutung durch Gefäßengstellung der wiederum eine reaktive Hyperämie folgt, eine Entzündungshemmung sowie eine generelle Abschwellung des betroffenen Gewebes.

Die ursprünglichste Form der äußerlichen Kühlung ist dabei die lokale Anwendung von Eis (ca. -20 °C) von 5 Min. bis max. 20 Min. (z.B. an den Gelenken). In der modernen Medizin gibt es aber auch Ganzkörperkältetherapien in trockener Luft (ca. -180 °C) für die Dauer von 2 Min. (z.B. bei Rheuma) oder als Eistauchbad (ca. 10 °C).

Durch Wassereis in Form von Eiswürfeln oder Eisbeuteln, bei dessen Schmelzen sich eine Temperatur von 0 °C einstellt, lässt sich eine Anästhesie oder zumindest eine deutliche Reduzierung des Schmerzempfindens erreichen.

Kälteanästhesie ist eine Form der örtlichen Betäubung, bei der man die betreffenden Partien durch Kälte schmerzunempfindlich macht. Man verwendet dabei z.B. festes Kohlendioxid oder Ethylchlorid; Letzteres meist in Form eines Sprays, dem sogenannten Eisspray. Eissprays werden oft bei Sportverletzungen oder beim Piercen, aber auch bei starkem Rheuma zur Kryotherapie eingesetzt.

Eisspray ist ein in einer Sprühdose abgefülltes Flüssiggas, mit dem Gegenstände oder Körperteile auf Temperaturen bis zu -55 °C abgekühlt werden können. Verschiedene Gase und Gasgemische werden angewendet, wobei als Unterscheidungskriterien hauptsächlich die tiefste erreichbare Temperatur und die Brennbarkeit in Frage kommen. U. a. werden entzündliche Kohlenwasserstoffe wie Propan, Butan, Pentan oder Mischungen aus diesen Stoffen verwendet. In der Medizin kommt vorwiegend das ebenfalls brennbare Chlorethan zum Einsatz.

EP 404334 A1 offenbart eine wirkstoffhaltige Aerosolkomposition bestehen aus einem Gemisch von (i) einer organischen Substanz mit einem Siedepunkt zwischen 10 °C und 40 °C und (ii) einer organischen Substanz mit einem Siedepunkt zwischen -45 °C und -10 °C.

Substanzen für (i) können dabei z.B. Isopentan, Methylpentan oder Diethylether sein, für (II) z.B. Propan/Butan-Mischungen oder Dimethylether sein. In diese Aerosolzubereitungen können dann pharmazeutische Wirkstoffe gelöst und zur Anwendung gebracht werden.

Nachteilig bei den vorstehenden Formen der Kälteanästhesie mit Eissprays ist jedoch, dass sie bei Anwendung an Menschen aufgrund der schwierigen exakten Dosierbarkeit erhebliche, zum Teil auch irreversible Gewebeschäden hervorrufen können.

Weiterhin nachteilig ist, dass die schmerzlindernde Kühlwirkung dieser Art Aerosole nicht von langanhaltender Natur ist, da die natürliche Hautdurchblutung nach Beendigung der direkten Sprayanwendung für ein sofortiges Verdunsten der Gase und somit wieder für die Herstellung der normalen Hauttemperatur im betroffenen Areal sorgt.

WO 2005032522 A1 beschreibt einen Aerosol-Schaum enthaltend pharmazeutische Wirkstoffe in einer Öl-in-Wasser Emulsion zur topischen Anwendung. In dieser Anwendungsform kann die Kühlwirkung eines Aerosols deutlich moderater gestaltet werden, insbesondere durch die geforderte Abwesenheit von flüchtigen niederen Alkoholen.

Aus ökologischen Gesichtspunkten sehr nachteilig an diesem Aerosol ist allerdings die geforderte ausschließliche Nutzung von Hydroflouralkanen als Treibmittel für diese Art von Aerosolen.

Die üblicherweise in der Medizin zur Linderung muskuloskelettaler Schmerzzustände angewendeten kühlenden Schmerzgele wie z.B. Voltaren®, Emulgel®, Dolgit Mikrogel oder Dolobene® Ibu Gel enthalten in der Regel pharmazeutische Wirkstoffe zur topischen Anwendung wie nichtsteroidale Antirheumatika (NSAR), z.B. Diclofenac oder Ibuprofen. Die Wirkstoffe müssen dabei nach Auftragen durch die Hautschichten penetrieren um zu ihrem Wirkort zu gelangen und eine beginnende Schmerzlinderung zu entfalten, dieser Vorgang kann dabei je nach persönlichem Hauttyp 15 bis 60 Minuten benötigen.

EP 704206 A1 offenbart eine pharmazeutische Zusammensetzung mit mindestens einem systemisch und/oder lokal wirkenden topisch applizierbaren Wirkstoff. Die Zusammensetzung weist eine solche flüssige Konsistenz auf, dass sie als Tröpfchen versprühbar ist und dass die Zusammensetzung nach dem Versprühen innerhalb kürzester Zeit, weniger als 4 Sekunden, auf der Haut eine solche Zubereitung ausbildet, die im Vergleich zu der ursprünglichen flüssigen Zusammensetzung eine deutlich höhere Konzentration an dem Wirkstoff aufweist. Die pharmazeutische Zusammensetzung umfasst eine Dispersion, Lösung und/oder eine Dispersion , die als Flüssigkeit bzw. Flüssigkeitsgemisch ein pharmazeutisch unbedenkliches organisches Lösemittel bzw. Lösemittelgemisch und/oder Wasser enthält. Das organische Lösemittel kann Alkohol oder ein Alkoholgemisch umfassen, der bzw. das leicht verdampfbar ist. Weiterhin kann die pharmazeutische Zubereitung mindestens einen Gelbildner, bevorzugt ein Phospholipid bzw. ein Phospholipidgemisch, enthalten, mit dem die Zusammensetzung nach dem Versprühen innerhalb kürzester Zeit auf der Körperoberfläche eine gelartige Zubereitung ausbildet. Zur Anwendung für diese pharmazeutischen Zubereitungen kommen aufgrund der notwendigen Tröpfchengrößenverteilung Pumpzerstäuber in Frage.

Eine weitere Darreichungsform bevorzugt aus einem Pumpzerstäuber wird in EP 206291 A2 offenbart. Hier umfasst die Zubereitung eine Phenylalkansäure als Wirkstoff. Des Weiteren enthält sie ein Lösungsmittelgemisch aus a) einem oder mehreren flüchtigen und b) einem oder mehreren nichtflüchtigen Lösungsmitteln. Als flüchtige Lösungsmittel sind Ethanol, Propanol oder Isopropanol allein oder als Gemisch definiert. Als nichtflüchtige Lösungsmittel werden mehrwertige Alkohole wie z.B. Propylenglykol und Glycerin definiert.

Die physikalische Kühlwirkung und damit verbunden unmittelbare Schmerzlinderung vorstehend beschriebener pharmazeutischer Gele wird dabei hauptsächlich durch Verdunstung des Hauptinhaltsstoffes Wasser, gelegentlich in Kombination mit ein oder mehrwertigen Alkoholen und oder ätherischen Ölen bzw. Menthol, hervorgerufen und von den Betroffenen meist nur als gering empfunden.

Aufgabe der vorliegenden Erfindung ist es daher ein wirkstoffhaltiges topisches Kühlgel zur Darreichung als halbfeste Form aus Tuben oder auch als Aerosolspray herzustellen, welches nach Auftragen auf das betroffene Hautareal durch physikalische Verdunstung bei Verreibung eine signifikante Temperaturabsenkung und damit verbunden eine für den Anwender direkt spürbare Schmerzlinderung hervorruft, bei gleichzeitiger Minimierung des Risikos von durch Kälte verursachten Gewebeschäden.

Im Stand der Technik, wie DE 100 65 046 A1, US 2009/ 0209642 A1 oder WO 2008/ 070368 A2, werden verschiedene Verdicker, Polymere, Lösemittel und Alkohole für topisch applizierbare Zubereitungen offenbart, die jedoch keinen Weg zur vorliegenden Erfindung nahelegen.

Die Erfindung ist ein Gel oder eine Mikroemulsion umfassend Wasser, Diethylether und Ammonium Acryloyldimethyltaurat/Vinylpyrrolidon Copolymer.

Ein Gel wird in der Regel als ein feindisperses System aus mindestens einer festen und einer flüssigen Phase definiert. Die feste Phase bildet dabei ein schwammartiges, dreidimensionales Netzwerk, dessen Poren durch eine Flüssigkeit (*Lyogel*) oder ein Gas (*Xerogel*) ausgefüllt sind. Ist das Netzwerk porös und Luft oder Treibmittel das eingelagerte Gas, so wird das Gel auch als *Aerogel* oder Aerosolgel bezeichnet.

Diethylether zeichnet sich durch einen niedrigen Siedepunkt und damit verbunden durch eine schnelle Verdunstung und gute Kühlwirkung auf der Haut aus. Andererseits ist Diethylether nicht mit Wasser mischbar, so dass es bei Zugabe von Diethylether zu wässrigen Emulsionen oder Gelen zu einem sofortigen Zusammenbruch der Emulsions- oder Gelstrukturen und damit zu einer Phasenseparation kommt.

Ein analoger Zusammenbruch der Matrixstrukturen zeigt sich wenn man wässrige Emulsionen oder Gele zur Anwendung als Aerosol herstellen will und dabei als Treibgas Dimethylether (DME) einsetzt.

Durch die erfindungsgemäße Herstellung von Mikroemulsionen oder Gelen unter Einsatz von Ammonium Acryloyldimethyltaurat/Vinylpyrrolidon Copolymer als Gelbildungsagens bleiben die ursprünglichen Matrixstrukturen der wässrigen Emulsionen und Gele überraschend auch bei Zugabe von Diethylether bzw. Verwendung von Dimethylether als Treibgas vollständig erhalten.

In diesem Falle wirkt der Gelbildner als Phasenvermittler zwischen den nicht mischbaren, sich separierenden Stoffen. Wobei das Quellverhalten des Verdickers für eine Einlagerung der wässrigen und nicht wässrigen Bestandteile der Matrix sorgt. Durch diese Einlagerung wird ein direkter Kontakt der nicht kompatiblen Moleküle verhindert, was einer Phasenseparierung entgegenwirkt.

Als Gelbildungsagens wird Ammonium Acryloyldimethyltaurat/Vinylpyrrolidon Copolymer (z.B. Aristoflex AVC von Clariant Ltd, UK) zugesetzt.

Um die starke Kühlwirkung hinsichtlich Temperatur und Wirkdauer der erfindungsgemäßen Zubereitung als Aerosol herzustellen muss eine Kombination aus Diethylether als leichtflüchtigem, kühlendem Excipient und Dimethylether (DME) als gasförmigem Treibmittel verwendet werden. Der Vorteil dieser Kombination liegt in der sofortigen Verdampfung des Treibmittels DME und der daraus resultierenden subjektiv starken Sofortkühlwirkung und dem langsamer verdampfenden Diethylether der zu einer Ausweitung der Kühldauer beiträgt.

Es wird somit ein Sofortkühleffekt als auch ein länger andauernder Kühleffekt erzielt.

Vorteilhaft im Sinne der vorliegenden Erfindung ist dabei ein Anteil von 0,1 - 30 Gew.% Diethylether in der Gelzubereitung zu zusetzen, bezogen auf die Gesamtmasse der Zubereitung ohne Treibmittel. Die Zubereitungen lassen sich auch mit höherem Diethyletheranteil (bis 30%) drucklos in Tuben abfüllen und liegen als gebrauchsfertiges Gel vor.

Die Zubereitungen können alternativ auch als Aerosole bereit gestellt und verwendet werden.

Die Zubereitung umfasst im Wesentlichen nur ein Treibmittel, Dimethylether. Das Treibmittel ist in der Zusammensetzung des erfindungsgemäßen Produktes in einer Menge von 5 bis 50 Gew.%, besonders bevorzugt von 10 bis 35 Gew.%, bezogen auf die Gesamtzusammensetzung incl. des Treibmittels, enthalten. Sind lediglich geringe Mengen (<5%) anderer Treibmittel, in Spuren oder eingeschleppt, in der Aerosolgelzubereitung enthalten, so gilt die Gelzubereitung weiterhin als nur mit einem einzigen Treibmittel versehen.

Der Wasseranteil des erfindungsgemäßen Gels liegt vorteilhaft im Bereich von etwa 25 bis 70 Gew.%, insbesondere 20 bis 70 Gew.%, vorteilhaft im Bereich von 40 bis 55 Gew.%, bezogen auf die Gesamtmasse des Gels ohne Treibmittel.

Neben der Zubereitung als Gel ist es auch möglich eine von der Wirkung her analoge Zubereitung als Mikroemulsion herzustellen. Auch diese Formulierungen können alternativ als Aerosol oder als drucklose Zubereitung in einer Tube hergestellt werden.

Die Formulierungen der vorliegenden Erfindungen können mit einer breiten Palette an Wirkstoffen hergestellt werden. In der folgenden Tabelle sind Beispiele an zu zusetzenden Wirkstoffen genannt, ohne jedoch den Anspruch auf Vollständigkeit zu erheben.

| **Indikation:** | **Wirkstoff** |
|---|---|
| Antiseptika | Thymol |
| | Eugenol |
| | Triclosan |
| | Hexachlorophen |
| | Benzalkoniumchlorid |
| | Clioquinol |
| | Chinolinol |
| | Undecensäure |
| | Ethacridin |
| | Chlorhexidin |
| | Hexetidin |
| | Dodicin |
| | Iod |
| Nichtsteroidale Antirheumatika | Glykolsalicylat |
| | Flufenaminsäure |
| | Ibuprofen |
| | Etofenamat |
| | Ketoprofen |
| | Piroxicam |
| | Indomethacin |
| | Diclofenac-Natrium |
| | Capsaicin |
| | ethanolischer |
| | Capsicumextrakt |
| | Nonivamid |
| | Arnika Montana |
| Lokalanästhetika | Benzocain |
| | Lidocain |
| Terpenoide Wirkstoffe | Menthol |
| | Campher |
| | Pfefferminzöl |
| | Rosmarinöl |
| | Eukalyptusöl |

Die erfindungsgemäßen Zubereitungen umfassen vorteilhaft eine wässrige Phase (Gelphase), enthaltend Ammonium Acryloyldimethyltaurat/Vinylpyrrolidon Copolymer, und eine nicht wässrige Phase (Lipidphase) enthaltend ein oder mehrere Lipide.

Des Weiteren ggf. eine Wirkstoffphase, die zusammengefügt mit der Gel- und Lipidphase das Gel bzw. Mikroemulsion bilden.

Die kosmetischen oder dermatologischen Zubereitungen gemäß der Erfindung können ferner kosmetische Hilfsstoffe und weitere Wirkstoffe enthalten, wie sie üblicherweise in solchen Zubereitungen verwendet werden, z. B. Substanzen zum Verhindern des Schäumens, Farbstoffe und Farbpigmente, Verdickungsmittel, anfeuchtende und/oder feuchthaltende Substanzen, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösungsmittel oder Silikonderivate, Selbstbräuner, Puffer, pH Regulatoren, Pflanzliche Extrakte, Puder, Sebumabsorbierende Substanzen, UV-Filter, Wirkstoffe wie zum Beispiel Anti Age, Anti-Cellulite, Anti Akne, Anti-Rosacea, Anti-Neurodermitis, Antioxidantien, Moisturiser, Chelatbildner, Antitraspirantien, Bleich und Färbemittel etc. sofern der Zusatz die geforderten Eigenschaften hinsichtlich der Gelstabilität, Kühleffekte und Hautverträglichkeit nicht behindert.

Nachfolgende Beispiele erläutern die erfindungsgemäßen Gele bzw. Mikroemulsionen. Die Prozenzangaben beziehen sich auf Massenprozent bezogen auf die Gesamtmasse der Zubereitung ohne Treibmittel.

### Beispiel 1:

### Herstellung Gelphase:

2g Aristoflex AVC und 2g Sorbitol werden in Pulverform miteinander vermischt. 50% des resultierenden Gemisches werden unter kontinuierlichem Rühren in 60g Wasser eingerührt und auf 80°C erhitzt. Innerhalb von 3 Stunden werden die übrigen 50% des Gemisches unter starkem Rühren zur Wasserphase hinzugegeben.

### Herstellung Lipidphase:

Eine Mischung von 32g Isoceteth 20, 12g Glyceryl Isostearat und 16g Coco-Caprylate/Caprate wird im Wasserbad auf 95°C erhitzt.

### Herstellung Wirkstoffphase:

10g Ibuprofen und 2g Menthol werden bei Raumtemperatur durch Rühren in 64g Propylenglycol gelöst.

### Herstellung der fertigen Zubereitung:

Die Gelphase wird in der Rührschüssel einer KitchenAid Maschine vorgelegt. Bei starkem Rühren fügt man die Lipidphase und die Wirkstoffphase zu der Gelphase hinzu und rührt die Mischung 60min bei langsamer Rührgeschwindigkeit. Während der Abkühlung auf Raumtemperatur bildet sich die Mikroemulsion aus.

Die Mikroemulsion wird anschließend unter Rühren mit der vorgesehenen Menge an Diethylether (5 - 30 Gew.%) versetzt und in Tuben, bzw. in Aerosoldosen zur Begasung abgefüllt. Die Aerosoldosen werden mit Dimethylether begast.

### Beispiel 2:

### Herstellung Gelphase:

In einer Rührschüssel einer KitchenAid Maschine werden 61g Wasser vorgelegt und unter starkem Rühren 5g Aristoflex AVC in Portionen über einen Zeitraum von 10 min zugegeben. Die resultierende Mischung wird unter starkem Rühren weitere 15min gequollen.

### Herstellung Wirkstoffphase:

In einem Becherglas werden 98g Ethanol Pharm. Eur., 21g Propylenglycol und 4g Glycerin vorgelegt und unter Rühren vermischt. Zu dieser Mischung fügt man 10g Ibuprofen und 1g Menthol hinzu und löst die Wirkstoffe unter Rühren bei Raumtemperatur homogen auf.

### Herstellung der fertigen Zubereitung:

Die Wirkstoffphase wird unter kontinuierlichem Rühren zu der Gelphase in der KitchenAid Maschine zugegeben. Die resultierende Mischung wird unter Rühren 30min homogenisiert.

Das Gel wird anschließend unter Rühren mit der vorgesehenen Menge an Diethylether (5 - 30%) versetzt und in Tuben, bzw. in Aerosoldosen zur Begasung abgefüllt. Die Aerosoldosen werden mit Dimethylether begast.

### Beispiel 3:

### Herstellung Gelphase:

In einer Rührschüssel einer KitchenAid Maschine werden 63,7g Wasser vorgelegt und unter starkem Rühren 5,3g Aristoflex AVC in Portionen über einen Zeitraum von 10 min zugegeben. Die resultierende Mischung wird unter starkem Rühren weitere 15min gequollen.

### Herstellung Wirkstoffphase:

In einem Becherglas werden 103,6g Ethanol Pharm. Eur., 22,1g Propylenglycol und 4,2g Glycerin vorgelegt und unter Rühren vermischt. Zu dieser Mischung fügt man 0,063g Capsaicin und 1,037g Menthol hinzu und löst die Wirkstoffe unter Rühren bei Raumtemperatur homogen auf.

### Herstellung der fertigen Zubereitung:

Die Wirkstoffphase wird unter kontinuierlichem Rühren zu der Gelphase in der KitchenAid Maschine zugegeben. Die resultierende Mischung wird unter Rühren 30min homogenisiert.

Das Gel wird anschließend unter Rühren mit der vorgesehenen Menge an Diethylether (5 - 30%) versetzt und in Tuben, bzw. in Aerosoldosen zur Begasung abgefüllt. Die Aerosoldosen werden mit Dimethylether begast.

### Beispiel 4:

### Herstellung Gelphase:

In einer Rührschüssel einer KitchenAid Maschine werden 63,0g Wasser vorgelegt und unter starkem Rühren 5,3g Aristoflex AVC in Portionen über einen Zeitraum von 10 min zugegeben. Die resultierende Mischung wird unter starkem Rühren weitere 15min gequollen.

### Herstellung Wirkstoffphase:

In einem Becherglas werden 103,6g Ethanol Pharm. Eur., 22,1g Propylenglycol und 4,0g Glycerin vorgelegt und unter Rühren vermischt. Zu dieser Mischung fügt man 1,0 Vanillylbutylether und 1,0g Menthol hinzu und löst die Wirkstoffe unter Rühren bei Raumtemperatur homogen auf.

### Herstellung der fertigen Zubereitung:

Die Wirkstoffphase wird unter kontinuierlichem Rühren zu der Gelphase in der KitchenAid Maschine zugegeben. Die resultierende Mischung wird unter Rühren 30min homogenisiert.

Das Gel wird anschließend unter Rühren mit der vorgesehenen Menge an Diethylether (5 - 30%) versetzt und in Tuben, bzw. in Aerosoldosen zur Begasung abgefüllt. Die Aerosoldosen werden mit Dimethylether begast.

### Beispiel 5:

### Herstellung Gelphase:

In einer Rührschüssel einer KitchenAid Maschine werden 63g Wasser vorgelegt und unter starkem Rühren 5g Aristoflex AVC in Portionen über einen Zeitraum von 10 min zugegeben. Die resultierende Mischung wird unter starkem Rühren weitere 15min gequollen.

### Herstellung Wirkstoffphase:

In einem Becherglas werden 100g Ethanol Pharm. Eur., 21g Propylenglycol und 4g Glycerin vorgelegt und unter Rühren vermischt. Zu dieser Mischung fügt man 6g Arnikaextrakt und 1g Menthol hinzu und löst die Wirkstoffe unter Rühren bei Raumtemperatur homogen auf.

### Herstellung der fertigen Zubereitung:

Die Wirkstoffphase wird unter kontinuierlichem Rühren zu der Gelphase in der KitchenAid Maschine zugegeben. Die resultierende Mischung wird unter Rühren 30min homogenisiert.

Das Gel wird anschließend unter Rühren mit der vorgesehenen Menge an Diethylether (5 - 30%) versetzt und in Tuben, bzw. in Aerosoldosen zur Begasung abgefüllt. Die Aerosoldosen werden mit Dimethylether begast.

## Patentansprüche

1. Zubereitung in Form eines Gels oder Mikroemulsion umfassend Wasser, Diethylether und Ammonium Acryloyldimethyltaurat/Vinylpyrrolidon Copolymer.

2. Zubereitung nach Anspruch 1 **dadurch gekennzeichnet, dass** der Anteil an Diethylether im Bereich von 0,1 - 35 Gew.%, bezogen auf die Gesamtmasse der Zubereitung ohne Treibmittel gewählt wird.

3. Zubereitung nach einem der vorstehenden Ansprüche in Form eines Aerosols enthaltend Dimethylether als Treibmittel.

4. Zubereitung nach einem der vorstehenden Ansprüche umfassend ein oder mehrere Wirkstoffe, bevorzugt pharmazeutische und/oder kosmetische Wirkstoffe.

5. Zubereitung nach einem der vorstehenden Ansprüche umfassend eine wässrige Phase (Gelphase), enthaltend Ammonium Acryloyldimethyltaurat/Vinylpyrrolidon Copolymer, und eine nicht wässrige Phase (Lipidphase) enthaltend ein oder mehrere Lipide.

6. Zubereitung nach einem der vorstehenden Ansprüche in der Darreichungsform Dispenser oder Tube.

## Claims

1. Preparation in the form of a gel or microemulsion comprising water, diethyl ether and ammonium acryloyldimethyltaurate/vinylpyrrolidone copolymer.

2. Preparation according to Claim 1, **characterized in that** the proportion of diethyl ether is selected within the range of 0.1-35% by weight, based on the total mass of the preparation without propellant.

3. Preparation according to either of the preceding claims in the form of an aerosol containing dimethyl ether as propellant.

4. Preparation according to any of the preceding claims comprising one or more active ingredients, preferably pharmaceutical and/or cosmetic active ingredients.

5. Preparation according to any of the preceding claims comprising an aqueous phase (gel phase), containing ammonium acryloyldimethyltaurate/vinylpyrrolidone copolymer, and a non-aqueous phase (lipid phase) containing one or more lipids.

6. Preparation according to any of the preceding claims in a dispenser or tube administration form.

## Revendications

1. Préparation sous la forme d'un gel ou d'une microémulsion comprenant de l'eau, de l'éther diéthylique et un copolymère d'acryloyldiméthyltaurate d'ammonium/vinylpyrrolidone.

2. Préparation selon la revendication 1, **caractérisée en ce que** la proportion d'éther diéthylique est choisie dans la plage allant de 0,1 à 35 % en poids, par rapport à la masse totale de la préparation sans agent gonflant.

3. Préparation selon l'une quelconque des revendications précédentes, sous la forme d'un aérosol contenant de l'éther diméthylique en tant qu'agent gonflant.

4. Préparation selon l'une quelconque des revendications précédentes, comprenant un ou plusieurs agents actifs, de préférence des agents actifs pharmaceutiques et/ou cosmétiques.

5. Préparation selon l'une quelconque des revendications précédentes, comprenant une phase aqueuse (phase de gel), contenant le copolymère d'acryloyldiméthyltaurate d'ammonium/vinylpyrrolidone, et une phase non aqueuse (phase lipidique), contenant un ou plusieurs lipides.

6. Préparation selon l'une quelconque des revendications précédentes, sous la forme pharmaceutique distributeur ou tube.
